# EUROPEAN PATENT APPLICATION

(11) **EP 3 327 873 A1**
(43) Date of publication of application: **30.05.2018**
(21) Application number: 16200812.2
(22) Date of filing: 25.11.2016
(51) Int. Cl.: H01R 13/52

(54) **MEDICAL DEVICE PLUG**

(71) Applicant: Roche Diabetes Care GmbH, 68305 Mannheim (DE); F. Hoffmann-La Roche AG, 4070 Basel (CH)
(72) Inventor: Kube, Oliver, 67549 Worms (DE); Schramm, Rene, 69118 Heidelberg (DE); Abril, Sara, 4054 Basel (CH)
(74) Representative: Richardt Patentanwälte PartG mbB

(57) **Abstract**

The invention provides for a medical device plug (100) configured for mating with a medical device socket (102) and for forming at least one electrical connection (114) with the medical device socket. The medical device plug comprises an insertion portion (108) with a distal end (110). The distal end is configured for inserting into the medical device socket along an axis (116). The insertion portion comprises a sealing element (104). The sealing element is configured for forming a seal of the at least one electrical connection at the distal end when the medical device plug is mated with the medical device socket. The medical device plug further comprises at least one fluid inflow restriction element (118, 120, 200, 300, 702, 1400, 1700) configured for reducing the inflow of fluid into the the medical device plug as the medical device plug is removed from the medical device socket.

## Description

### Field of the invention

The invention relates to medical devices, in particular the sealing of electrical connections against fluids.

### Background and related art

Many types of medical instruments have electrical connections between instrumentation such as controllers and various types of sensors. Water or bodily fluids may degrade or destroy these electrical connections. This may in turn cause the medical instrument to fail.

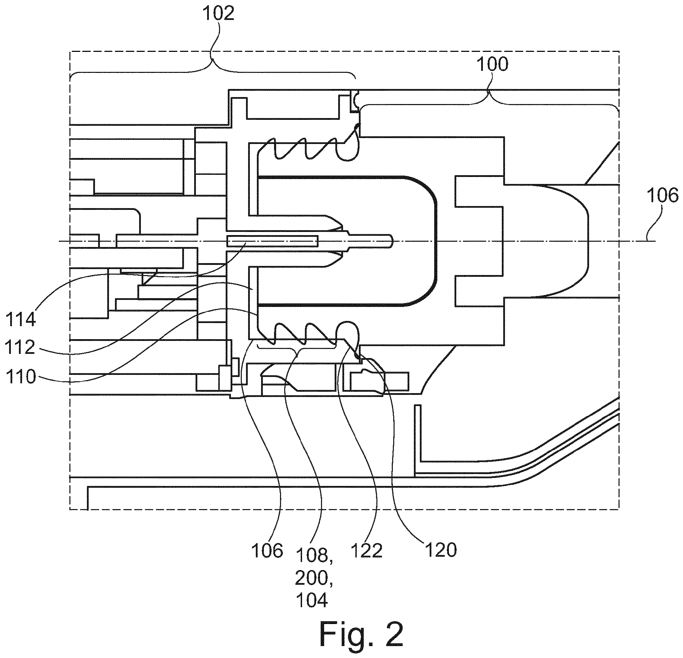

United States patent 8,506, 585 B2 discloses an insertion system having a base unit for placing on the body of a patient and an insertion device that can be coupled to the base unit, wherein the insertion device comprises an insertion needle holder for holding an insertion needle and a drive mechanism for displacing the insertion needle holder in a pricking direction. The insertion device comprises a locking mechanism causing locking of the drive mechanism in an active state and being set to an inactive state in which the locking is released by coupling the insertion device to the base unit. Fig. 2 illustrates an example of an electrical connector to connect the sensor with a transmitter.

### Summary

The invention provides for a medical device plug, a medical device socket, a continuous glucose monitoring sensor assembly, and a glucose monitoring system in the independent claims. Embodiments are given in the dependent claims.

When forming a connection between a plug and a socket of a medical device, sealing elements such as o-rings or other elastomeric elements may be used. The use of single o-rings or even double o-rings is common. When the plug and socket are compressed together they may form a watertight and/or airtight seal. Pusing the connectors together may form a sealed volume which encloses any electrical connections. Pushing the plug and the socket together has the effect of compressing air within the sealed volume as the two are pressed together. Because the pressure within the sealed volume increases, some of the air from the sealed volume may escape as the two are pressed together. Also, because the pressure is elevated some air may possibly leak out over time.

When the plug and socket are pulled apart, the air that leaked out or escaped causes the opposite effect: a vacuum may be formed as the plug and socket are pulled apart. For medical devices that are used repeatedly this may be detrimental. Fluids such as water, cleaning agents, or even bodily fluids may be in the vicinity of the plug and socket. As the plug and socket are pulled apart this vacuum may suck the fluid into the connector. The fluid could possibly ruin or degrade electrical connections within the plug or socket and prevent reuse or degrade the performance of the medical device.

Embodiments of the invention may reduce or prevent the inflow of fluid into a medical device plug and/or medical device socket by using a fluid inflow restriction element. The fluid inflow restriction element may for example be a wiper, flap, or other element which may be used to reduce fluid inflow from a medical device plug and/or medical device socket and/or to remove fluid from a medical device plug and/or medical device socket during removal of the medical device plug from the medical device socket..

In one aspect, the invention provides for a medical device plug configured for mating with a medical device socket and for forming at least one electrical connection with the medical device socket. The medical device plug comprises an insertion portion with a distal end. The distal end is configured for inserting into the medical device socket along an axis. The insertion portion comprises a sealing element. The sealing element is configured for forming a seal of at least one electrical connection at the distal end when the medical device plug is mated with the medical device socket. The medical device plug further comprises at least one fluid inflow restriction element configured for reducing the inflow of fluid into the medical device plug as the medical device plug is removed from the medical device socket.

This embodiment may be beneficial because typically when medical device plugs are removed from medical device sockets the sealing elements may cause an inflow of fluid into the medical device socket or the distal end of the medical device plug. This may cause the medical device plug and/or the medical device socket to become ruined or contaminated with the fluid. Adding a fluid inflow restriction element may reduce the amount of fluid which may reduce damage in the medical device plug and/or the medical device socket.

In another embodiment, the insertion portion is cylindrically symmetric about the axis.

In another embodiment, the at least one electrical connection are is located at the distal end.

In another embodiment, the sealing element is an O-ring.

In another embodiment, the sealing element may comprise a resilient sealing material that can be radially compressed about the axis.

In another embodiment, the sealing element comprises several sealing element or sub-sealing elements that are useful in sealing the plug to the medical device socket along the axis.

In another embodiment, when the seal is formed the medical device plug and the medical device socket form a sealed volume. The sealed volume may for instance be water tight or air tight. The distal end and the electrical connection are within the sealed volume.

In another embodiment, the at least one fluid inflow restriction element comprises a sealing lip configured for contacting an outer surface of the medical device socket when the medical device socket and the medical device plug are mated. This may be beneficial because the sealing lip may help to keep liquid or other fluid that splashes onto the medical device plug from entering and contacting the sealing element. This may further reduce the inflow of fluid into the medical device plug. The sealing lip may in some examples for instance be made of an elastic or elastomeric material that contacts the medical device plug and may help to protect the medical device plug from fluid.

In another embodiment, the at least one fluid inflow restriction element comprises a first wiper element configured for wiping the fluid from the medical device socket as the medical device plug is removed from the medical device socket. The first wiper element is between the sealing element and the distal end. The first wiper element is configured for allowing air to pass between the wiper element and the medical device socket.

This embodiment may be beneficial because the wiper element allows air to enter the volume between the first wiper element and the sealing element. A common cause of fluid inflow into a device and plug combination is that when the plug is removed a vacuum can be created which serves to draw fluid into the plug or socket. Having a wiper immediately behind the sealing element as it is removed may serve to wipe or push any fluid out of the medical device plug or medical device socket. Having a means for air to pass between the wiper element and the medical device socket may reduce the likelihood that a vacuum will develop adjacent to the sealing element.

In another embodiment, the medical device plug comprises an air channel between the distal end of the medical device plug and an intermediate region between the first wiper element and the sealing element. This embodiment may be beneficial because it may help to eliminate a large vacuum adjacent to the sealing element while at the same time the first wiper element is able to remove any fluid which does enter into the medical device plug or socket.

In another embodiment, the at least one fluid inflow restriction element comprises a second wiper element configured for wiping the fluid from the medical device socket as the medical device plug is removed from the medical device socket. The sealing element is between the distal end and the second wiper element. The second wiper element is configured for allowing air to pass between the wiper element and the medical device socket. In this embodiment there is a wiper before and after the sealing element. The first and second wiper elements may help serve to further reduce the amount of fluid that is trapped and then enters into the medical device plug or medical device socket as the medical device plug is removed from the medical device socket.

In another embodiment, the at least one fluid inflow restriction element comprises the sealing element. The sealing element comprises multiple sealing ridges. The multiple sealing ridges have a saw tooth profile along the direction of the axis. This embodiment may be beneficial because the multiple sealing ridges may serve to both seal the medical device plug to the medical device socket as well as help to remove any fluid that does enter.

In another embodiment, the at least one fluid inflow restriction element comprises the sealing element. The sealing element comprises multiple sealing ridges. The multiple sealing ridges are formed as a portion of a conical helix. The portion of the conical helix is narrower at the distal end. The sealing ridges furthest from the distal end seal the best as they have a larger diameter. The use of the conical helix for the sealing ridges may be beneficial because it may confine fluid that enters into the medical device plug and socket to a region that is furthest from the distal end.

In another embodiment, the at least one fluid inflow restriction element comprises a sponge. The use of a sponge may be beneficial because it may both serve to directly absorb fluid which enters into the plug and device socket pair and also the sponge, when more full with fluid, may serve as a wiper to physically remove fluid as the medical device plug is removed from the medical device socket.

In another embodiment, the sponge is between the distal end and the sealing element. The sponge is configured for wiping the fluid from the medical device socket and/or absorbing fluid as the medical device plug is removed from the medical device socket. In this embodiment the sponge is within the sealed volume. As the medical device plug is removed from the medical device socket fluid may possibly enter. The sponge will prevent this by firstly directly absorbing the fluid and secondly by wiping the fluid from the medical device socket.

In another embodiment, the sealing element is between the distal end and the sponge. In this example the sponge is outside of any volume that is sealed by the sealing element. This embodiment may be beneficial because the sponge may serve to absorb fluid that splashes onto the medical device plug and it may also serve to shield the medical device plug from fluid.

In another embodiment, the seal is any one of the following: the seal is water tight, the seal is a hermetic seal, the seal is air tight, the seal is waterproof, the seal is configured for sealing fluids, and the seal is configured for sealing body fluids.

In another aspect, the invention provides for a medical device socket which is configured for receiving the medical device plug according to an embodiment. This embodiment may be beneficial because the combination of the medical device socket and plug may be useful for providing a plug and socket combination that is sealed effectively against body fluids or other fluids and reduces the amount of fluid that enters into the medical device socket or plug. This may increase the useful lifespan of the medical device plug and/or socket.

In another embodiment, the medical device socket comprises a capillary region configured for capturing the fluid. This may be beneficial because if fluid does enter into the medical device socket the capillary region may be able to capture the fluid before it reaches an electrical connection. This may reduce the chance that the medical device socket fails due to contamination by the fluid.

In another aspect, the invention provides for a continuous glucose monitoring sensor assembly comprising the medical device plug of an embodiment. A continuous glucose monitoring sensor may for example be a sensor which is inserted subcutaneously into a subject and is used for monitoring the level of glucose on a repeated or continuous basis.

In another embodiment, the invention provides for a medical device electrical sealing system. For example, this may comprise of the medical device socket and the medical device plug according to an embodiment.

In another aspect, the invention provides for a glucose monitoring system comprising the continuous glucose monitoring sensor assembly according to an embodiment. The glucose monitoring system further comprises an electronic portion. The electronic portion comprises a medical device socket configured for receiving the medical device plug. This embodiment may be beneficial during repeated use of the glucose monitoring system because it may provide for an increased lifespan of the glucose monitoring system due to a reduced amount of contamination of the medical device plug and/or the medical device socket. This embodiment may also be beneficial when the glucose monitoring system is used a single time. During single use the medical device socket and the medical device plug may provide for better protection of the electrical connection which may make the glucose monitoring system more robust.

### Brief description of the drawings

In the following embodiments of the invention are explained in greater detail, by way of example only, making reference to the drawings in which:
- Fig. 1: illustrates an example of a medical device plug and a medical device socket;
- Fig. 2: illustrates a further example of a medical device plug and a medical device socket;
- Fig. 3: illustrates a seal between a further example of a medical device plug and a medical device socket;
- Fig. 4: illustrates a further example of a medical device plug;
- Fig. 5: illustrates a further example of a medical device plug;
- Fig. 6: illustrates a further example of a medical device plug and a medical device socket;
- Fig. 7: illustrates a seal between a further example of a medical device plug and a medical device socket;
- Fig. 8: illustrates a seal between a further example of a medical device plug and a medical device socket;
- Fig. 9: illustrates a seal between a further example of a medical device plug and a medical device socket;
- Fig. 10: illustrates a seal between a further example of a medical device plug and a medical device socket;
- Fig. 11: illustrates a seal between a further example of a medical device plug and a medical device socket;
- Fig. 12: illustrates a seal between a further example of a medical device plug and a medical device socket;
- Fig. 13: illustrates a seal between a further example of a medical device plug and a medical device socket;
- Fig. 14: illustrates a seal between a further example of a medical device plug and a medical device socket;
- Fig. 15: illustrates a seal between a further example of a medical device plug and a medical device socket;
- Fig. 16: illustrates a seal between a further example of a medical device plug and a medical device socket;
- Fig. 17: illustrates a seal between a further example of a medical device plug and a medical device socket; and
- Fig. 18: illustrates a further example of a medical device plug and a medical device socket.

### Detailed Description

Like numbered elements in these figures are either equivalent elements or perform the same function. Elements which have been discussed previously will not necessarily be discussed in later figures if the function is equivalent.

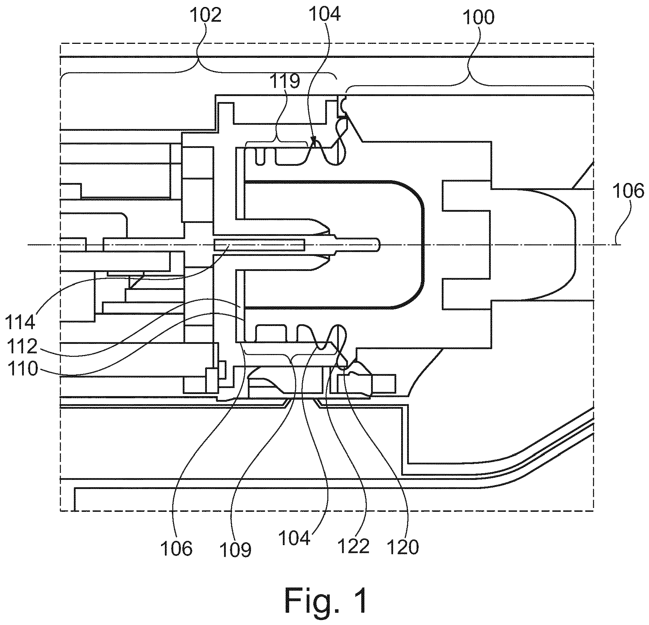

Fig. 1 shows an example of a medical device socket 102 and a medical device plug 100. The medical device plug 100 comprises a sealing element 104 that seals against a receptacle 106 of the medical device plug 100. The medical device plug 100 comprises an insertion portion 108 that is inserted into the receptacle 106. The insertion portion 108 has a distal end 110. The combination of the receptacle 106 and the sealing element 104 form a sealed volume 112. The sealed volume encompasses the distal end 110 and also an electrical connection 114 that is formed between the medical device socket 102 and the medical device plug 100. In this particular example the receptacle 106 has a cylindrical symmetry about the axis 106. The insertion portion 108 is inserted into the receptacle 106 or the plug 100 along the insertion axis 106.

The medical device socket 102 is shown as further comprising a sealing lip 120. The sealing lip 120 contacts an outer surface 122 of the medical device plug 100. The sealing lip 120 in this example is a flap or lip of elastic or elastomeric material that helps to prevent fluid from splashing against or contacting the sealing element 104. This may help reduce the amount of fluid which enters into the sealed volume 112 as the medical device socket 102 is removed from the medical device plug 100. The conical helix 118 does not seal in the way that the sealing element 104 does. However, as the medical device socket 102 is removed from the medical device plug the conical helix 118 may serve to wipe or pull fluid that may enter into the sealed volume 112.

Fig. 2 shows a further example of a medical device socket 102 and medical device plug 100 as an assembly. The example shown in Fig. 2 is similar to the example shown in Fig. 1 except the sealing element 104 and the fluid inflow restriction element 200 are different. In this case the sealing element 104 is combined with the fluid inflow restriction element 200. In this case the combination is a number of multiple sealing ridges 200 that have a saw tooth profile along the axis 106. This embodiment may be beneficial because the saw tooth profile provides for multiple seals and also the saw-like tooth profile has a tendency that each saw tooth forms its own sealed volume and also serves to pull or wipe fluid from and away from the sealed volume 112.

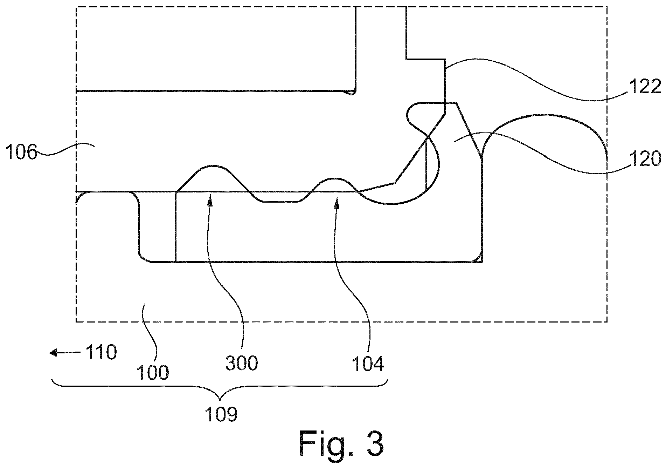

Fig. 3 shows a close up of the insertion portion 108 of the medical device socket 102 against the receptacle 106. In this example there is a sealing element 104 and an additional sealing element 300 which functions as the fluid inflow restriction element. This Fig. shows the sealing lip 120 in greater detail. The sealing lip 120 can be shown as contacting an outer surface 122. The sealing lip 120 may help to keep fluid from splashing near or in the vicinity of the sealing element 104.

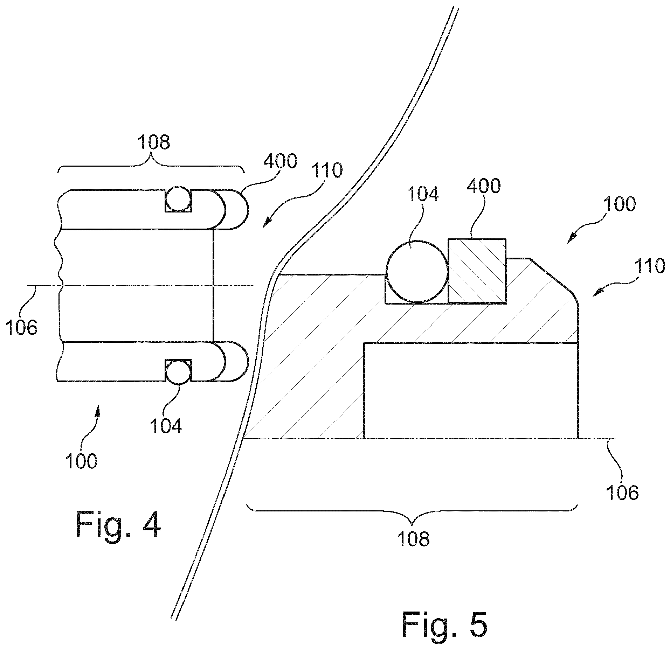

Fig. 4 shows a further example of a medical device socket 102. In this example there is a sponge 400 which is mounted at the distal end 110. The sponge 400 may serve as a fluid inflow restriction element. The sponge may serve to directly absorb liquid which enters into the socket 102 or it may serve to wipe water from the socket as the plug 100 is removed.

Fig. 5 shows an alternative mechanical arrangement to that shown in Fig. 4. In this example the sponge 400 is mounted directly adjacent to the sealing element 104. The sponge 400 is closer to the distal end 110 than the sealing element 104 is. This example may function particularly well as when fluid is able to pass past the sealing element 104 it is immediately absorbed or wiped by the sponge 400.

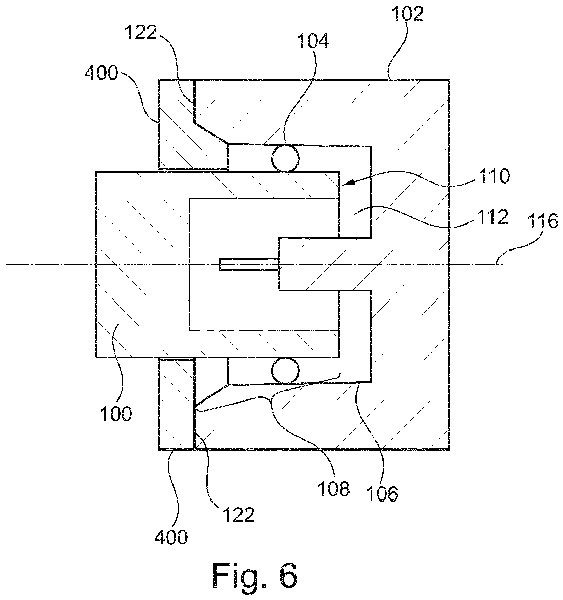

Fig. 6 shows an example of a medical device plug 100 and a medical device socket 102. The medical device plug 100 is similar to that shown in Figs. 4 and 5 except the sponge 400 is mounted further away from the distal end 110 than the sealing element 104 is. The sponge 400 is shown as contacting an outer surface 122 of the medical device socket 102. The sponge 400 in this case helps to prevent fluid from splashing against the sealing element 104. This helps to prevent fluid from passing the sealing element 104 as the medical device plug 100 is removed from the medical device socket 102.

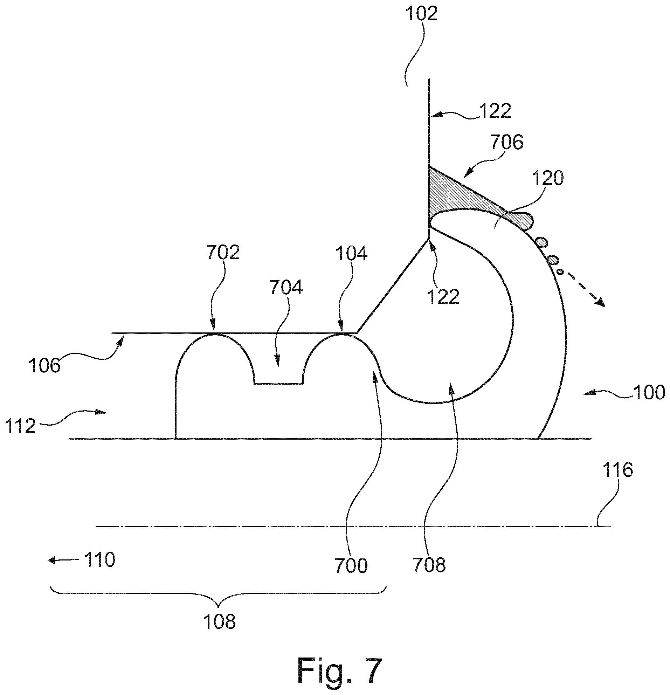

Fig. 7 illustrates the region where the insertion portion 108 is inserted into the receptacle 106. In this example there is a sealing element 104 which forms a first barrier 700 against fluid. Closer to the distal end there is a wiper 702. The wiper in this example does not seal completely as the sealing element 104 does. This creates a region of mutual pressure 704. This avoids having a high vacuum adjacent to the sealing element 104 as it is removed from the receptacle 106. To further reduce the amount of fluid 706 there is a sealing lip 120 that contacts an outer surface 122 of the medical device socket 102. The sealing lip 120 helps prevent fluid 706 from splashing against or entering the sealing element 104. Additionally there is a cavity 708 which serves to trap any fluid 706 which may make it past the sealing lip 120.

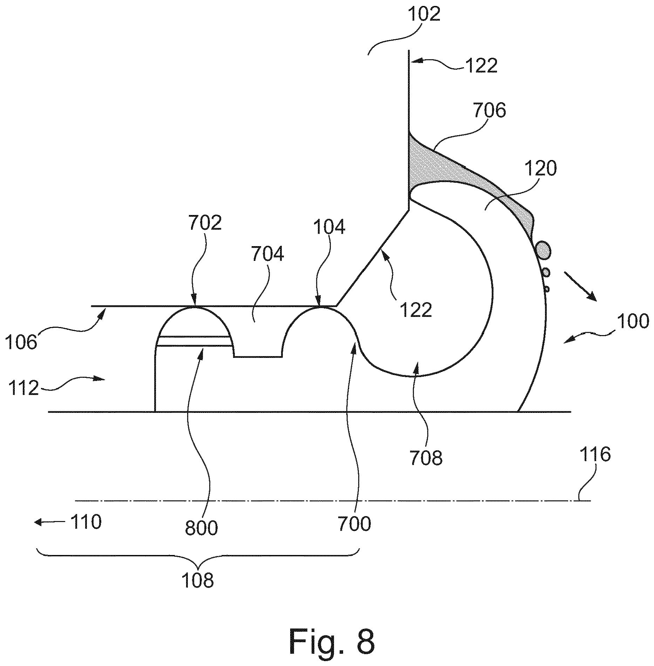

Fig. 8 shows an example similar to that illustrated in Fig. 7. The example in Fig. 8 is the same as in Fig. 7 except the wiper 702 additionally has an air channel 800 which serves as a direct vent between the neutral pressure zone 704 and the sealed volume 112. This may serve to help reduce the pressure in the region directly adjacent to the sealing element 104 as it is pulled from the medical device socket 102.

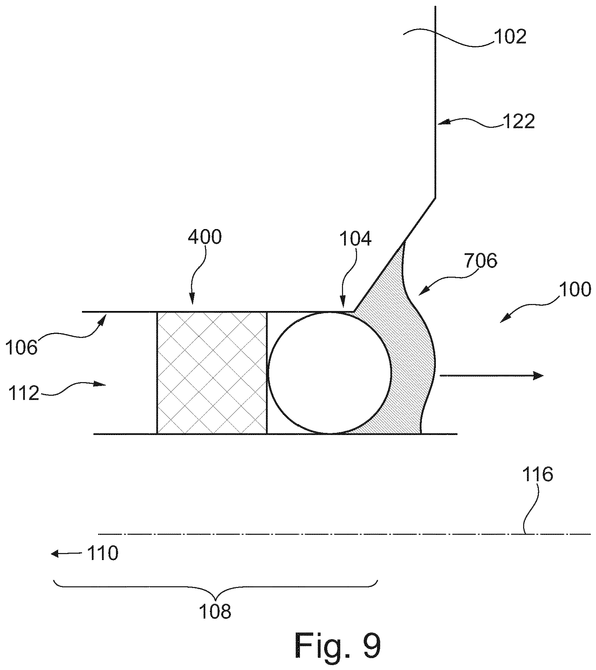

Fig. 9 shows an enlarged region where the insertion portion 108 has been inserted into the receptacle 106. The example shown in Fig. 9 is similar to that of Fig. 5. There is a sponge 400 which absorbs water before it could enter the sealed volume 112 and also may serve as a wiper to remove any fluid 706 that gets past the sealing element 104 as it is removed from the medical device socket 102.

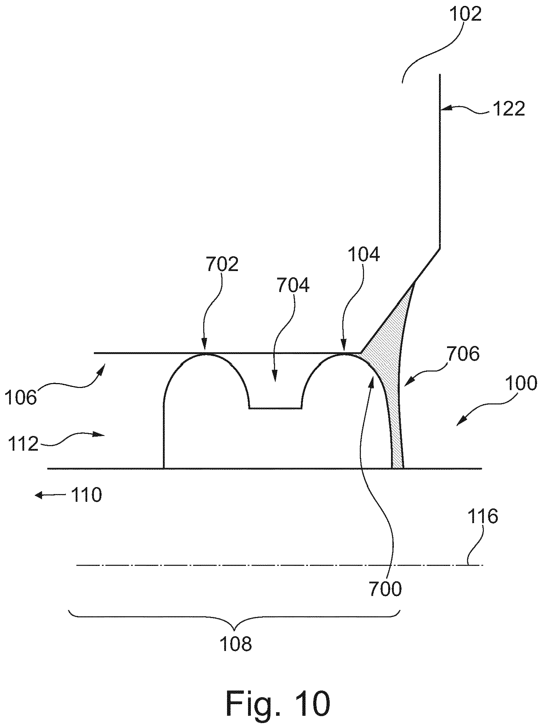

Fig. 10 shows an example that is similar to the example shown in Fig. 7 except the sealing lip 120 is absent in Fig. 10.

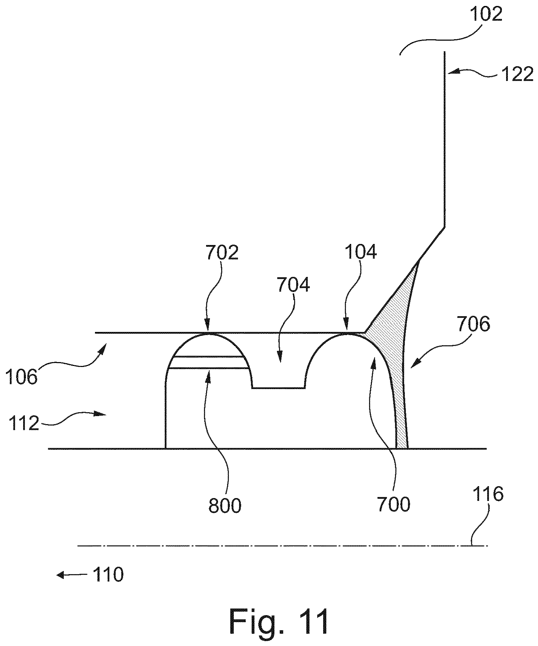

Fig. 11 illustrates an example that is similar to that that is shown in Fig. 8. The example in Fig. 11 is identical of that of Fig. 8 except the sealing lip 120 is absent.

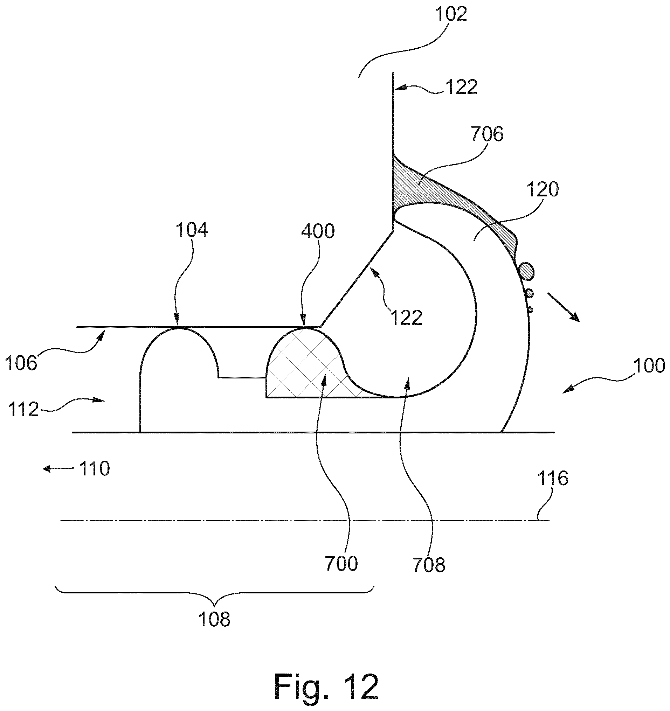

Fig. 12 illustrates an example similar to that shown in Fig. 7 with several differences. In the example in Fig. 12 the sealing element 104 is located closer to the distal end 110. The sealing element 104 is between the distal end 110 and a sponge or foam. The sponge or foam 400 serves as a first barrier against fluid 706. The sponge may absorb water during use and may help prevent it from entering into the sealed volume 112.

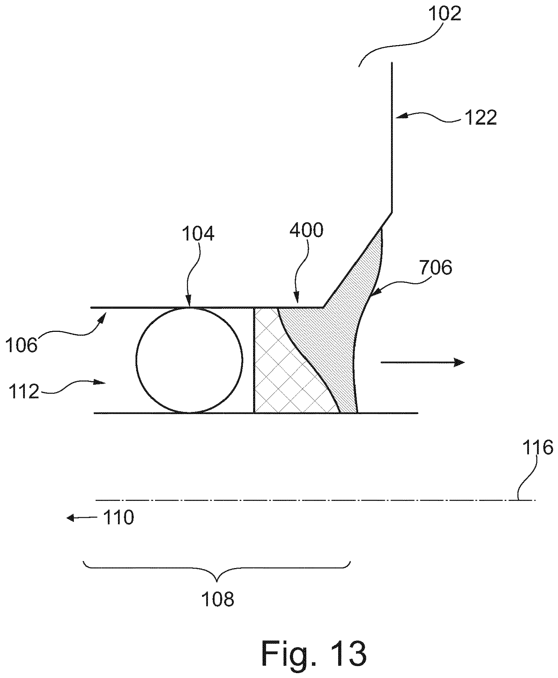

Fig. 13 illustrates an example that is similar to that that is shown in Fig. 12. However, in this example the sealing lip 120 is absent. The sealing element 104 is also illustrated as being a portion of an O-ring. However, the sealing element 104 in Fig. 13 has the same function as the sealing element 104 in Fig. 12.

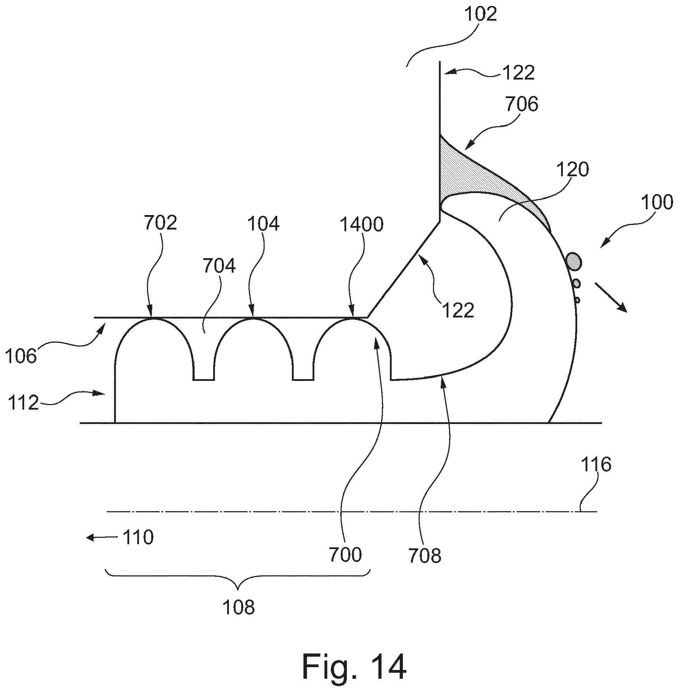

Fig. 14 shows a further example which is similar to the example shown in Fig. 7. However, in the example illustrated in Fig. 14 there is an additional wiper 1400. The additional wiper 1400 has been added such that the sealing element 104 is closer to the distal end 110 than the second wiper 1400. The second wiper 1400 is similar in construction to the first wiper 702 in that it does not provide a completely air or water tight seal. The function of the wiper 1400 is to wipe fluid away from the sealing element 104 as the medical device plug 100 is removed from the medical device socket 102. The example in Fig. 14 then has a wiper 1400, 702 before and after the sealing element 104.

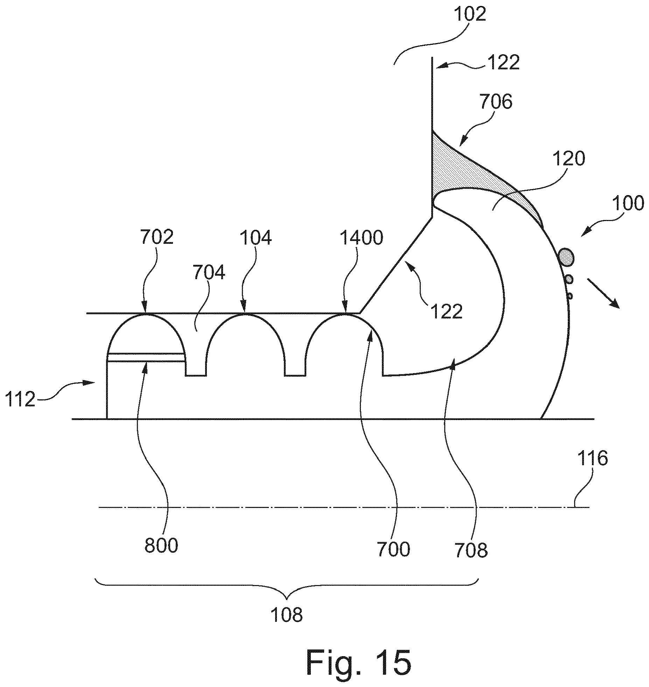

Fig. 15 shows a further example which combines the features of Fig. 14 and Fig. 8. The wiper 702 contains an air channel 800 as is illustrated in Fig. 8.

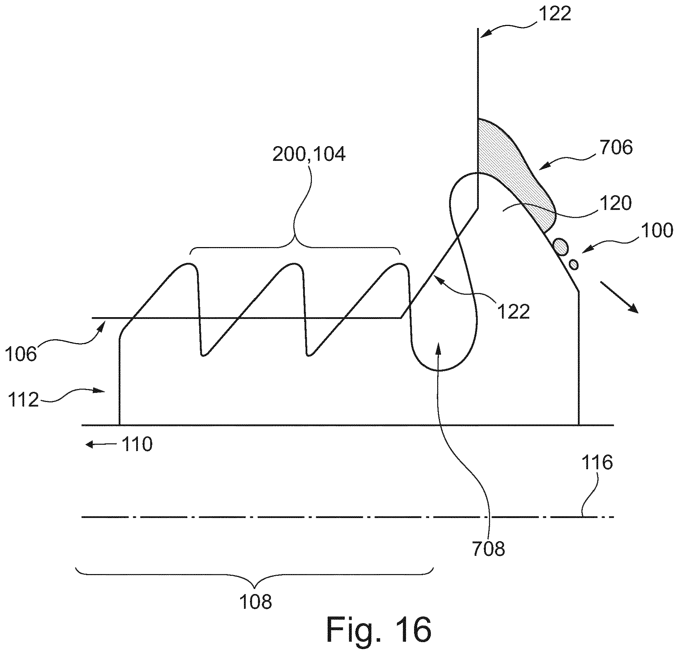

Fig. 16 illustrates the saw tooth-shaped multiple sealing ridges 200 of Fig. 2 in greater detail. Fig. 16 shows the region of Fig. 2 enlarged where the insertion portion 108 contacts the receptacle 106. The saw tooth-shaped multiple sealing ridges 200 and the sealing lip 120 are shown as overlapping with the outer surface 122 or the receptacle 106. This is intended to indicate regions that are compressed against either the receptacle 106 or the outer surface 122.

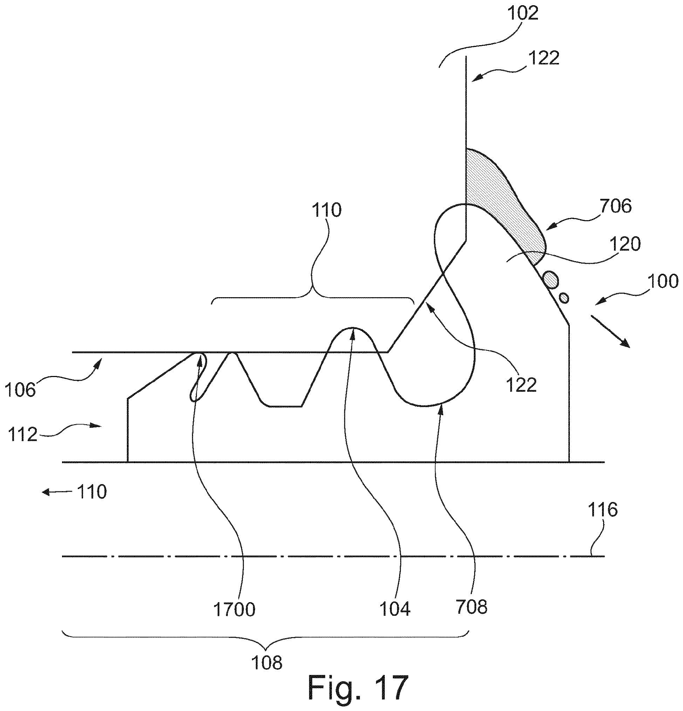

Fig. 17 illustrates the conical helix 118 of Fig. 1 where it contacts the receptacle 106 in greater detail. As with Fig. 16, overlapping regions between the sealing element 104 or the sealing lip 120 that overlap with either the outer surface 122 or the receptacle 106 are intended to represent compression. In this Fig. it can be seen that the conical helix 118 also comprises at the end a wiper 1700 that helps to remove fluid 706 from the medical device socket 102.

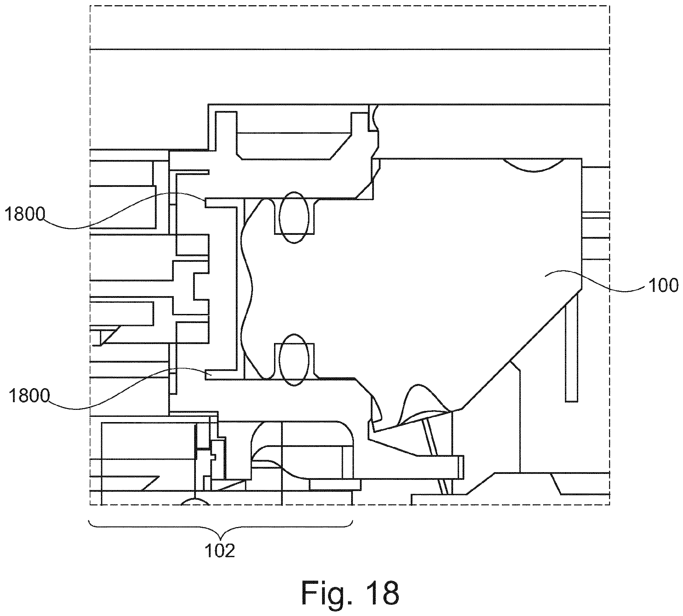

Fig. 18 illustrates a modification of the medical device socket 102 that may be used with any of the previously illustrated examples. In this example the socket 1800 has a region of capillaries 1800. The capillary region may for instance be small slits or grooves which have a dimension small enough that capillary action of the fluid draws fluid into the capillary region 1800. The capillary region may be useful in capturing fluid which enters into the medical device socket 102. The capillary region 1800 may serve to hold fluid and keep it away from any electrical connections.

### List of reference numerals

- 100: medical device plug
- 102: medical device socket
- 104: sealing element
- 106: receptacle
- 108: insertion portion
- 110: distal end
- 112: sealed volume
- 114: electrical connection
- 116: axis
- 118: conical helix
- 120: sealing lip
- 122: outer surface
- 200: saw tooth shaped multiple sealing ridges
- 300: additional sealing element
- 400: sponge or foam
- 700: first barrier
- 702: wiper
- 704: neutral pressure zone
- 706: fluid
- 708: cavity
- 800: air channel
- 1400: wiper
- 1700: wiper
- 1800: capillary region

## Claims

1. A medical device plug (100) configured for mating with a medical device socket (102) and for forming at least one electrical connection (114) with the medical device socket, wherein the medical device plug (100) comprises an insertion portion (108) with a distal end (110), wherein the distal end (110) is configured for inserting into the medical device socket (102) along an axis (116), wherein the insertion portion (108) comprises a sealing element (104), wherein the sealing element (104) is configured for forming a seal of the at least one electrical connection (114) at the distal end (110) when the medical device plug (100) is mated with the medical device socket (102), wherein the medical device plug (100) further comprises at least one fluid inflow restriction element (118, 120, 200, 300, 702, 1400, 1700) configured for reducing the inflow of fluid into the the medical device plug (100) as the medical device plug (100) is removed from the medical device socket (102).

2. The medical device plug (100) of any one of claim 1, wherein the at least one fluid inflow restriction element comprises a sealing lip (120) configured for contacting an outer surface of the medical device socket (102) when the medical device socket (102) and the medical device plug (100) are mated.

3. The medical device plug (100) of claim 1 or 2, wherein the at least one fluid inflow restriction element comprises a first wiper element (702) configured for wiping the fluid from the medical device socket (102) as the medical device plug (100) is removed from the medical device socket (102), wherein the first wiper element (702) is between the sealing element (104) and the distal end (110), and wherein the first wiper element (702) is configured for allowing air to pass between the wiper element and the medical device socket (102).

4. The medical device plug (100) of any one of claim 3, wherein the medical device plug (100) comprises an air channel (800) between the distal end (110) of the medical device plug (100) and an intermediate region (704) between the first wiper element (702) and the sealing element (104).

5. The medical device plug (100) of any one of the preceding claims, wherein the at least one fluid inflow restriction element comprises a second wiper element (1400) configured for wiping the fluid from the medical device socket (102) as the medical device plug (100) is removed from the medical device socket (102), wherein the sealing element (104) is between the distal end (110) and the second wiper element (1400), and wherein the second wiper element is configured for allowing air to pass between the second wiper element (1400) and the medical device socket (102).

6. The medical device plug (100) of claim 1 or 2, wherein the at least one fluid inflow restriction element (200) comprises the sealing element (104), wherein the sealing element (104) comprises multiple sealing ridges, wherein the multiple sealing ridges have a saw tooth profile along the axis (116).

7. The medical device plug (100) of claim 1 or 2, wherein the at least one fluid inflow restriction element (118) comprises the sealing element (104), wherein the sealing element (104) comprises multiple sealing ridges, wherein the multiple sealing ridges are formed as as a portion of a conical helix, wherein the portion of the conical helix is narrower at the distal end (110).

8. The medical device plug (100) of any one of the preceding claims, wherein the at least one fluid inflow restriction element comprises a sponge (400).

9. The medical device plug (100) of any one of claim 8, wherein the sponge (400) is between the distal end (110) and the sealing element (104), wherein the sponge (400) is configured for wiping the fluid from the medical device socket (102) and/or absorbing fluid as the medical device plug (100) is removed from the medical device socket (102).

10. The medical device plug (100) of any one of claim 8, wherein the sealing element (104) is between the distal end (110) and the sponge (400).

11. The medical device plug (100) of any one of the preceding claims, wherein the seal is any one of the following: the seal is watertight, the seal is a hermetic seal, the seal is airtight, the seal is water proof, the seal is configured for sealing fluids, and the seal is configured for sealing body fluids.

12. A medical device socket (102) for receiving the medical device plug (100) of any one of the preceding claims.

13. The medical device socket (102) of claim 12, wherein the medical device socket (102) comprises a capillary region (1800) configured for capturing the fluid.

14. A continuous glucose monitoring sensor assembly comprising the medical device plug (100) of any one of claims 1 through 11.

15. A glucose monitoring system comprising the continuous glucose monitoring sensor assembly of claim 14, wherein the glucose monitoring system further comprises an electronic portion, wherein the electronic portion comprises a medical device socket (102) configured for receiving the medical device plug (100).
